# EUROPEAN PATENT SPECIFICATION

(11) **EP 1 443 114 B1**
(45) Date of publication and mention of the grant of the patent: **26.04.2006**
(21) Application number: 03733351.5
(22) Date of filing: 10.06.2003
(51) Int. Cl.: C12Q 1/02, C12N 5/08

(54) **METHOD OF CELL CULTURE**
ZELLKULTURVERFAHREN
PROCEDE DE CULTURE DE CELLULES

(30) Priority: 17.06.2002 JP 2002175658
(43) Date of publication of application: 04.08.2004
(73) Proprietor: Olympus Corporation, Tokyo 151-0072 (JP)
(72) Inventor: INOUE, Hikaru, Higashimurayama-shi, Tokyo 189-0025 (JP); IRIE, Hiroyuki, Akishima-shi, Tokyo 196-0024 (JP); HIBINO, Hiroki, Hachioji-shi, Tokyo 193-0811 (JP); KOYANAGI, Hideki, Hachioji-shi, Tokyo 193-0804 (JP)
(74) Representative: Wibbelmann, Jobst
(86) International application number: PCT/JP2003/007348
(87) International publication number: WO 2003/106700

(56) References cited:
- EP-A- 0 866 120
- EP-A- 1 167 517
- WO-A-00/77170
- WO-A-02/28996
- WO-A1-93/00420
- JP-A- 9 154 567
- JP-A- 2001 224 366
- JP-A- 2002 125 656
- FORTIER L A ET AL: "ISOLATION AND CHONDROCYTIC DIFFERENTIATION OF EQUINE BONE MARROW- DERIVED MESENCHYMAL STEM CELLS" AMERICAN JOURNAL OF VETERINARY RESEARCH, XX, XX, vol. 59, no. 9, September 1998 (1998-09), pages 1182-1187, XP000882113 ISSN: 0002-9645
- SAFA A R ET AL: "A SIMPLE METHOD FOR SCANNING ELECTRON MICROSCOPE PREPARATION OF CELLS GROWN IN MULTI WELL CULTURE PLATES" STAIN TECHNOLOGY, vol. 57, no. 2, 1982, pages 107-112, XP008035755 ISSN: 0038-9153
- MCALINDEN M G ET AL: "Comparison of cancellous bone-derived cell proliferation in autologous human and fetal bovine serum" CELL TRANSPLANTATION, vol. 9, no. 4, July 2000 (2000-07), pages 445-451, XP008035756 ISSN: 0963-6897

## Description

### Technical Field

The present invention relates to a cell culturing method that allows the growth status of cells for transplant to be accurately determined without directly contacting the cells.

### Background Art

The bone at sites of missing bone caused by the removal of bone tumors or trauma is currently repaired by replenishing the missing bone with a bone filler. However, in cases of osteoporosis and so forth when the bone gradually becomes brittle, or in cases when the site of the missing bone covers a wide area, it is difficult to resolve the problem with the method described above.

Therefore, it has become necessary in recent years to attempt a new procedure in which bone marrow is collected from the patient, mesenchymal stem cells contained in the collected bone marrow are artificially differentiated into osteoblasts, and after allowing the osteoblasts to adequately grow, they are again returned to the patient. In this case, since osteoblasts are grown from bone marrow collected from the patient himself and are subsequently returned to the same patient, bone formation can be activated without causing an immune reaction.

However, in the case of artificially growing osteoblasts in the manner described above, it is necessary to accurately determine whether or not the osteoblasts have achieved adequate growth. Since the osteoblasts are eventually returned to the patient's body, it is desirable to avoid contact with the cells as much as possible. However, since the growth status of osteoblasts typically cannot be assessed visually, their growth status must be assessed by manipulating them in some way into a sample.

### Disclosure of the Invention

In consideration of the aforementioned circumstances, the object of the present invention is to provide a cell culturing method that allows the growth status of cells for transplant to be accurately determined without directly contacting the cells.

In order to achieve the aforementioned object, the present invention provides a cell culturing method for producing transplant cells according to claim 1. The method comprises: a step in which cells for transplant and cells for examination are cultured under identical conditions; and a step in which the growth status of said cells for transplant is assessed by using said cells for examination.

Moreover, the present invention provides a cell culturing method, further comprising a step in which the cells for transplant and the cells for examination are separately housed in a culture vessel having partitions for separately housing a plurality of samples in the same vessel.

Moreover, the present invention provides a cell culturing method, further comprising a step in which the orders of the number of cells per unit volume of the cells for transplant and the cells for examination are made to be equal at the start of culturing.

According to the present invention, since cells for transplant and cells for examination are cultured in an isolated state, the cells for examination can be easily removed without contacting the cells for transplant even when removing the cells for examination from a vessel. Moreover, since the cells for transplant and the cells for examination are housed in the same culture vessel, the cells for transplant and the cells for examination can constantly be kept in the same environment by moving the aforementioned culture vessel.

### Brief Description of the Drawings

Fig. 1 is a drawing for providing a simple explanation of a summary of the cell culturing system.
Fig. 2 is a flow chart for explaining the culturing step carried out at culturing center 2.
Fig. 3 is a schematic drawing showing a culture vessel as described in an embodiment of the present invention.

### Best Mode for Carrying Out the Invention

The following provides an explanation of an embodiment of the present invention with reference to the drawings.

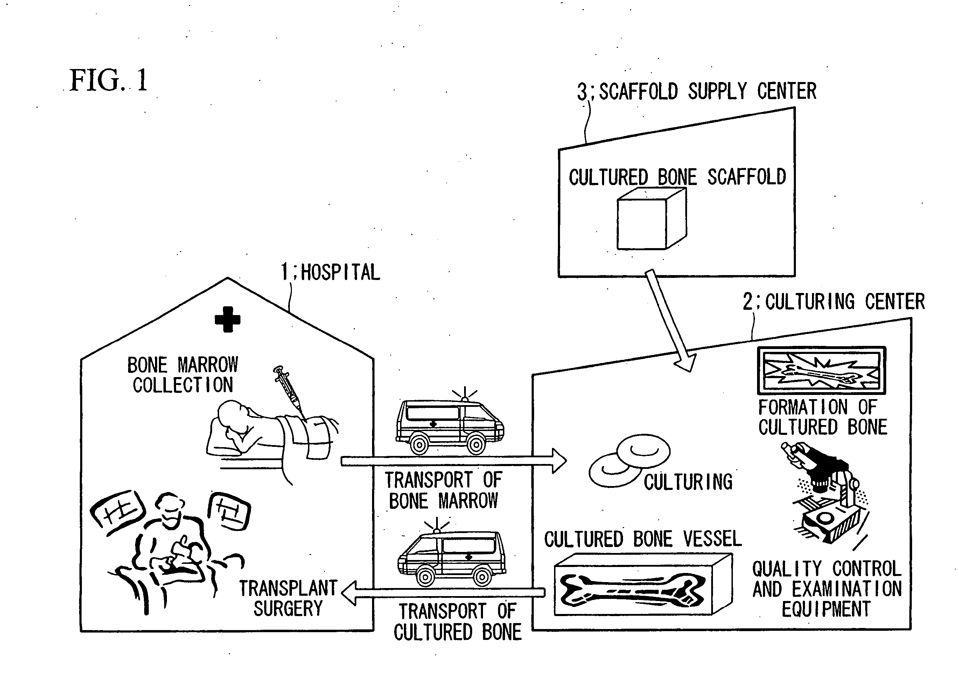

To begin with, a brief explanation is provided of a summary of the cell culturing system to which the cell culturing method as described in the present embodiment is applied with reference to Fig. 1.

In Fig. 1, bone marrow that has been collected from a patient at a hospital 1 is housed in a predetermined transport vessel and transported to a culturing center 2 to form the cultured bone. Furthermore, the transport vessel may be maintained at a temperature of about 37°C, a temperature of 4°C or frozen.

At culturing center 2, primary culturing in which mesenchymal stem cells contained in the transported bone marrow are cultured, secondary culturing in which cultured bone is formed by adding the cultured mesenchymal cells to a footing material referred to as a scaffold, and examinations and so forth for investigating whether or not bacteria and fungi are contained in the bone marrow and culture liquid, etc., are carried out, followed finally by housing the formed cultured bone in a predetermined transport vessel and transport to hospital 1. Furthermore, the transport vessel may be maintained at a temperature of about 37°C, a temperature of 4°C or frozen.

Furthermore, the scaffold used in secondary culturing is supplied from a scaffold supply center 3.

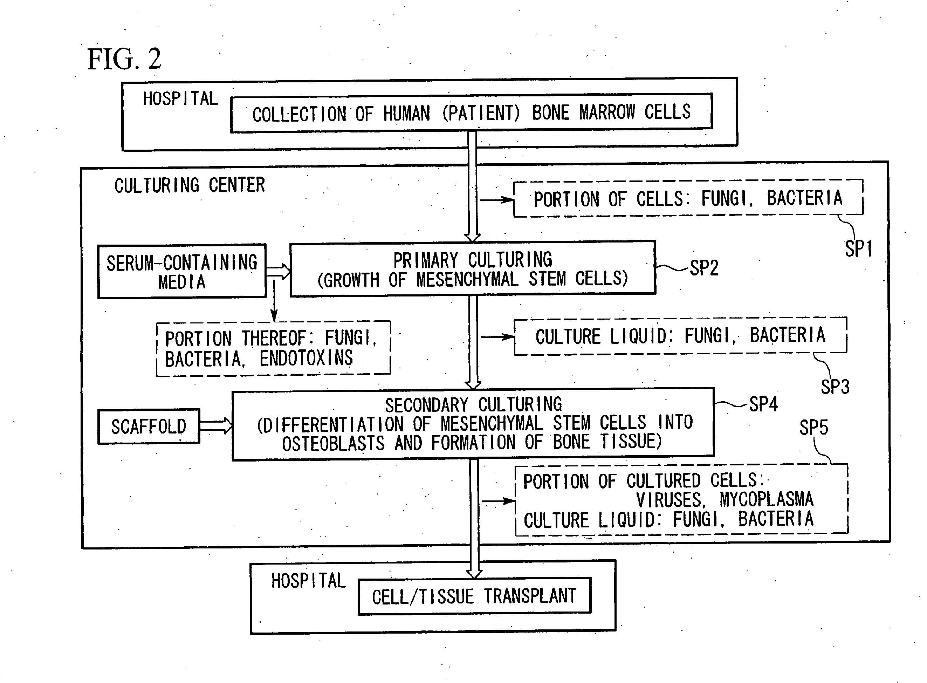

Next, a brief explanation is provided of the culturing step carried out at the culturing center 2 with reference to Fig. 2.

First, bone marrow that has been collected from a patient at hospital 1 is transported to the culturing center 2 housed in a predetermined transport vessel.

At culturing center 2, a portion of the bone marrow cells housed in the transport vessel is removed and examined to determine whether or not bacteria or fungi and so forth are contained in the collected bone marrow (Step SP1).

If there are no abnormalities found in the examination results, the bone marrow is transferred to a culturing step for growing mesenchymal stem cells contained in the bone marrow liquid (Step SP2). Furthermore, the culturing step for growing the mesenchymal stem cells is referred to as primary culturing. In this primary culturing, mesenchymal stem cells are grown by adding bone marrow that has been similarly examined in Step SP1 to media containing examined serum that has been confirmed to be free of fungi, bacteria, endotoxins and so forth (human or bovine serum is used in this media).

When the mesenchymal stem cells have grown to an adequate amount required to form cultured bone, they are next examined to determine whether or not they contain fungi, bacteria and so forth (Step SP3), and if there are no problems as a result of that examination, they are transferred to secondary culturing for formation of cultured bone (Step SP4).

In secondary culturing, the mesenchymal stem cells grown by primary culturing are adhered to a footing material referred to as a scaffold where they are differentiated into osteoblasts to form bone tissue (cultured bone).

This scaffold is a footing material composed of porous calcium phosphate in the manner of β-TCP. By adhering mesenchymal stem cells to this scaffold, differentiation from mesenchymal stem cells to osteoblasts is promoted resulting in the formation of bone tissue. Furthermore, growth factor and so forth may be added at this time to further accelerate differentiation to osteoblasts. Furthermore, a scaffold such as porous hydroxyapatite may also be used instead of the aforementioned β-TCP.

The cultured bone that has been formed in this manner is then transferred to step in which pre-transport examinations are performed such as examinations to determine whether or not the cultured bone contains viruses, mycoplasma, fungi or bacteria and so forth (Step SP5), and if there are no abnormalities, the cultured bone is transferred to a predetermined transport vessel and transported to hospital 1.

At the hospital that has received the cultured bone, transplant surgery is performed for transplanting said cultured bone into a patient on a preset surgery date.

Next, a detailed description is provided of the aforementioned secondary culturing.

First, in secondary culturing, mesenchymal stem cells grown by primary culturing are adhered to a scaffold for transplant and a scaffold for examination. In the following explanation, the cells adhered to the scaffold for transport are referred to as cells for transplant 200, while the cells adhered to the scaffold for examination are referred to as cells for examination 300. These cells for transplant 200 and cells for examination 300 are cultured under identical conditions. Scaffolds of the same size and shape are used for the scaffold for transplant and the scaffold for examination to which cells for transplant 200 and cells for examination 300 are respectively adhered in order to culture the cells under identical conditions, and cells for transplant 200 and cells for examination 300 are cultured in the same thermo-hygrostat using media of the same lot number. Moreover, cells for transplant 200 and cells for examination 300 are preferably adjusted so that the orders of the number of cells per unit volume are the same. More preferably, the cells for transplant 200 and cells for examination 300 are adjusted to 10⁶-10⁷ cells/cm³.

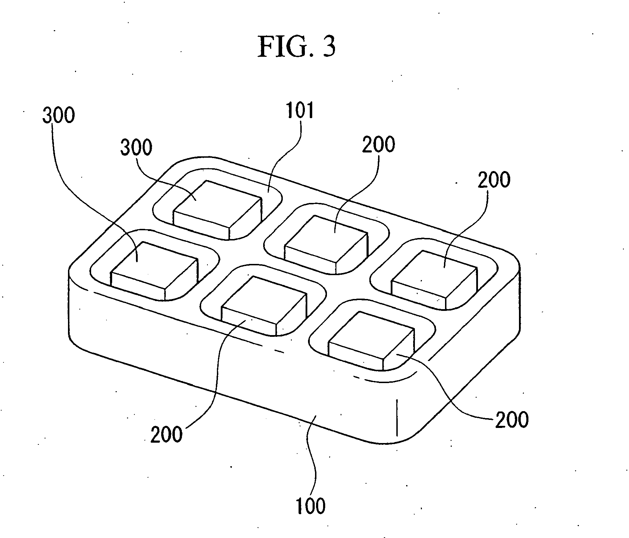

Next, these cells for transplant 200 and cells for examination 300 are respectively housed in culture vessel 100 shown in Fig. 3 together with the scaffold for transplant and the scaffold for examination. As shown in Fig. 3, culture vessel 100 is composed to have partitions so as to allow a plurality of samples to be separately housed within the same vessel. More specifically, samples are housed in a plurality of holes (indentations) formed by partitions, and the samples for transplant (containing cells for transplant 200 and the scaffold for transplant) and the samples for examination (containing cells for examination 300 and the scaffold for examination) are separately housed in these holes 101. In addition, this culture vessel 100 is preferably designed so as to be able to house about 3-50 samples for transplant and samples for examination.

Culture vessel 100, in which is housed cells for transplant 200 (sample for transplant) and cells for examination 300 (sample for examination), is then placed in the atmosphere in which secondary culturing is to be carried out, namely under conditions that are suitable for carrying out secondary culturing. For example, culture vessel 100 is placed in an atmosphere in which the temperature is held at 37±0.5°C and the carbon dioxide concentration is held at 5% by volume. As a result, the mesenchymal stem cells adhered to the scaffold phagocytize β-TCP and differentiates into osteoblasts, and bone tissue is formed in about 10 days to 2 weeks after the start of secondary culturing.

Next, cells for examination 300 housed in culture vessel 100 (sample for examination) are removed when about 2-30 days, and preferably 10 days, have elapsed from the start of secondary culturing. The growth status of osteoblasts in these cells for examination 300 is then investigated to confirm the formation of bone tissue, which is then used to assess the formation of bone tissue of cells for transplant 200.

For example, the following assessment techniques are examples of techniques for confirming the formation of bone tissue of cells for examination 300 during the aforementioned secondary culturing.

### (1) Assessment Based on Degree of Activity of Alkaline Phosphatase

Cells for examination are crushed, and the degree of activity of alkaline phosphatase of the crushed cells for examination 300 is measured. Since this alkaline phosphatase is a component that indicates the degree of activity of osteoblasts, measuring the degree of activity of this alkaline phosphatase makes it possible to determine the growth status of osteoblasts in secondary culturing, thereby allowing confirmation of the formation of bone tissue.

### (2) Assessment Using Microscopy

### (a)Assessment by Image Processing Using Images Obtained with a Confocal Microscope

Images of the surface of the scaffold for examination are obtained with a confocal microscope, and the osteoblasts are extracted by image processing. The growth status of the osteoblasts is then assessed by investigating whether or not the number of extracted cells exceeds a predetermined number to confirm the formation of bone tissue.

### (b)Assessment Using Ball Pen-Type Confocal Microscope

In the case the scaffold used has a cylindrical hole in the center, a ball pen-type confocal microscope is inserted into the cylindrical hole of the scaffold. The formation of bone tissue is then confirmed by confirming the presence of osteoblasts on the surface of the hole (inside the scaffold).

### (c)Assessment by Fluorescent Staining

After crushing cells for examination 300 and fluorescent staining the osteoblasts contained in the crushed cells for examination 300, the resulting fluorescence is measured. The result of the measurement, namely the growth status of osteoblasts corresponding to the number of fluorescent stained cells, is then assessed to confirm the formation of bone tissue. The aforementioned measurement of fluorescence can be carried out by counting the fluorescent stained osteoblasts using a microscope.

If the differentiation of mesenchymal stem cells into osteoblasts and the growth of the osteoblasts is able to be confirmed in cells for examination 300 by these assessment techniques, then the formation of bone tissue is judged to having progressed sufficiently. Cells for transplant 200 are then removed from culture vessel 100 and secondary culturing is completed. These cells for transplant 200 are then transferred to the following step in the form of the examination step shown in Fig. 2.

On the other hand, in the case the growth of osteoblasts in cells for examination 300 is not adequate, the remaining cells for transplant 200 and cells for examination 300 in culture vessel 100, in which the cells for examination 300 (sample for examination) used to assess secondary culturing were housed, are continued to be cultured. One of the cells for examination 300 (sample for examination) is again removed from culture vessel 100 after the passage of several days, and preferably about 3-7 days in consideration of formation of bone tissue, and the growth status of.osteoblasts in the removed cells for examination 300 is assessed.

If growth of osteoblasts is confirmed and the formation of bone tissue is adequate as a result, cells for transplant 200 are transferred to the examination step, while if formation of bone tissue is not adequate, secondary culturing is continued.

As has been explained above, according to the culturing method as described in the present embodiment, by culturing cells for transplant 200 and cells for examination 300 under identical conditions, the cell growth status of cells for examination 300 can be considered to be nearly identical to the cell growth status of cells for transplant 200. As a result, by confirming the cell growth status of cells for examination 300, the cell growth status of cells for transplant 200 can be determined without directly contacting cells for transplant 200.

In addition, since cells for transplant 200 (sample for transplant) and cells for examination 300 (sample for examination) are respectively and separately housed and cultured in culture vessel 100, which has partitions for separately housing a plurality of samples in a single vessel, each sample can be prevented from making contact. As a result, cells for examination 300 (sample for examination) can be easily removed from culture vessel 100 without contacting cells for transplant 200 even when they are removed from culture vessel 100. As a result, contamination of cells for transplant 200 by fungi and bacteria can be prevented when removing cells for examination 300 (sample for examination) from culture vessel 100. In addition, by placing culture vessel 100 housing both cells for transplant 200 and cells for examination 300 in an atmosphere suitable for secondary culturing at the start of secondary culturing, cells for transplant 200 and cells for examination 300 can easily be cultured under identical conditions.

Furthermore, although the case of primary culturing mesenchymal stem cells from bone marrow liquid, differentiating the mesenchymal stem cells into osteoblasts during secondary culturing and then growing these osteoblasts is described in the aforementioned embodiment, placental blood or peripheral blood and so forth may be collected instead of bone marrow liquid, and then primary culturing may then be carried out based on that sample. Alternatively, bone marrow liquid or placental blood and so forth may be directly inoculated onto a scaffold without undergoing primary culturing, and the mesenchymal stem cells may be grown on the scaffold and subsequently differentiated.

In addition, the cultured cells may also be somatic stem cells, ES cells, bone-related cells or chondrocytes and so forth in addition to mesenchymal stem cells. Moreover, the cells may be autologous cells or heterologous cells. In addition, stem cells may be transplanted directly without differentiating.

In addition, suitable growth factors, examples of which include BMP, FGF, TGF-β, VEGF, IGF, PDGF and HGF, may be added during culturing.

In addition, the following can be used instead of β-TCP during second culturing. Namely, any material be used provided it has affinity for body tissue, and material that can be absorbed by the body is even better. In addition, the material may also be porous. Examples of porous materials include porous ceramics having biocompatibility, collagen, polylactic acid or porous metals, and there are no limitations on such porous materials provided they have a large number of pores. In addition, calcium phosphate-based ceramics such as apatite and β-tricalcium phosphate (β-TCP), collagen or polylactic acid and so forth may also be used as porous materials. In addition, calcium-phosphate-based ceramics may be combined with collagen, or calcium phosphate-based ceramics may be combined with polylactic acid. β-tricalcium phosphate, collagen and polylactic acid have the characteristic of being biodegradable and absorbed by the body, while apatite has the characteristic of having high strength.

Furthermore, it goes without saying that a person with ordinary skill in the art would be able to suitably select and use an appropriate type of porous material corresponding to the transplant site and so forth.

Although the above has provided a detailed description of an embodiment of the present invention with reference to the drawings, concrete constitutions are not limited to this embodiment, but rather other designs and so forth are also included within a range that does not deviate from the purport of the invention.

### Industrial Applicability

As has been explained above, according to the cell culturing method of the present invention, since the growth status of cells for transplant can be assessed according to the growth status of cells for examination by culturing cells for transplant and cells for examination under identical conditions, the growth status of cells for transplant can be accurately confirmed without making any contact whatsoever with the cells for transplant. As a result, contamination of cells for transplant by fungi or bacteria and so forth can be avoided when assessing growth status.

In addition, according to the culturing method of the present invention, since cells for transplant and cells for examination are separately housed and cultured in a culture vessel capable of respectively and separately housing a plurality of samples, contact between each of the cells can be prevented and the cells can be easily removed even during removal of the cells for examination.

Moreover, by housing cells for transplant and cells for examination in the same culture vessel, and placing the culture vessel in an atmosphere suitable for secondary culturing at the start of secondary culturing, the cells for transplant and the cells for examination can easily be cultured under identical conditions. As a result, the amount of work required of workers engaged in secondary culturing can be reduced considerably.

## Claims

1. A method of producing transplant cells, comprising the steps of:
dividing a group of cells into transplant cells and examination cells;
culturing the transplant cells and the examination cells separately under identical conditions and in an isolated state; and
assessing the growth status of said transplant cells using said examination cells.

2. A method according to claim 1, further comprising the step of:
separately housing the transplant cells and the examination cells in a culture vessel having partitions for separately housing a plurality of samples in a single vessel.

3. A method according to claim 1, further comprising the step of:
making the orders of the number of cells per unit volume of the transplant cells and the examination cells to be equal at the start of culturing.

## Patentansprüche

1. Verfahren zur Herstellung von Transplantationszellen, aufweisend die Schritte:
Unterteilen einer Gruppe von Zellen in Transplantationszellen und Untersuchungszellen;
getrenntes Kultivieren der Transplantationszellen und der Untersuchungszellen unter identischen Bedingungen und in einem isolierten Zustand; und
Bestimmen des Wachstumszustandes der Transplantationszellen unter Verwendung der Untersuchungszellen.

2. Verfahren nach Anspruch 1, weiterhin mit dem Schritt:
separates Aufnehmen der Transplantationszellen und der Untersuchungszellen in einem Kulturgefäß, welches zur Aufnahme einer Mehrzahl von Proben in einem einzigen Gefäß Unterteilungen hat.

3. Verfahren nach Anspruch 1, weiterhin mit dem Schritt:
größenordnungsmäßiges Gleichmachen der Anzahl von Zellen pro Einheitsvolumen der Transplantationszellen und der Untersuchungszellen zu Beginn der Kultivierung.

## Revendications

1. Procédé pour la production de cellules à transplanter, comprenant les étapes de :
la division d'un groupe de cellules en cellules à transplanter et cellules à examiner ;
la culture des cellules à transplanter et des cellules à examiner séparément dans des conditions identiques de façon isolée ; et
l'évaluation de l'état de croissance desdites cellules à transplanter en utilisant lesdites cellules à examiner.

2. Procédé selon la revendication 1, comprenant en outre l'étape de :
la mise des cellules à transplanter et des cellules à examiner en logements séparés dans un récipient pour culture possédant des partitions permettant de placer plusieurs échantillons, séparément, dans un récipient unique.

3. Procédé selon la revendication 1, comprenant en outre l'étape de :
la mise à égalité des valeurs du nombre de cellules par unité de volume pour les cellules à transplanter et les cellules à examiner au début de la culture.
